(19) Europäisches Patentamt / European Patent Office / Office européen des brevets

(11) **EP 3 970 804 A1**

(12) **EUROPEAN PATENT APPLICATION**

(43) Date of publication:
**23.03.2022 Bulletin 2022/12**

(21) Application number: **20197604.0**

(22) Date of filing: **22.09.2020**

(51) International Patent Classification (IPC):
**A61Q 19/00** (2006.01) **A61P 17/10** (2006.01)
**A61P 17/00** (2006.01) **A61K 8/60** (2006.01)
**A61K 8/73** (2006.01) **A61K 8/365** (2006.01)
**A61K 8/42** (2006.01) **A61K 8/44** (2006.01)
**A61K 8/37** (2006.01) **A61K 8/92** (2006.01)
**A61K 31/19** (2006.01) **A61K 31/17** (2006.01)
**A61K 31/195** (2006.01) **A61K 31/215** (2006.01)
**A61K 31/7004** (2006.01) **A61K 31/7016** (2006.01)
**A61K 31/702** (2006.01)

(52) Cooperative Patent Classification (CPC):
(C-Sets available)
**A61Q 19/00; A61K 8/365; A61K 8/375; A61K 8/42;**
**A61K 8/44; A61K 8/60; A61K 8/73; A61K 8/92;**
**A61K 31/17; A61K 31/19; A61K 31/198;**
**A61K 31/22; A61K 31/7004; A61K 31/7016;**
**A61K 31/713;** (Cont.)

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Beiersdorf AG**
**20253 Hamburg (DE)**

(72) Inventor: **The designation of the inventor has not yet been filed**

(74) Representative: **Uexküll & Stolberg**
**Partnerschaft von**
**Patent- und Rechtsanwälten mbB**
**Beselerstraße 4**
**22607 Hamburg (DE)**

(54) **COMPOSITION FOR PROMOTING THE SKIN MICROBIOME**

(57) The present invention generally relates to the field of skin care. More particularly, the invention relates to a skin care composition that comprises growth-enhancing compounds which promote the development of a healthy skin microbiome. These growth-enhancing compounds are metabolized by the microorganisms of the skin microbiome after their topical application to the skin, thereby supporting growth of the microorganisms. The growth-enhancing compounds and compositions comprising same can be used for treating or preventing skin diseases, for promoting the development of a healthy skin microbiome, for stabilizing a healthy skin microbiome, and for promoting growth of bacteria of the skin microbiome.

Figure 3 A

**(Cont. next page)**

(52) Cooperative Patent Classification (CPC): (Cont.)
**A61P 17/00; A61P 17/10**

C-Sets
**A61K 31/17, A61K 2300/00;**
**A61K 31/19, A61K 2300/00;**
**A61K 31/198, A61K 2300/00;**
**A61K 31/22, A61K 2300/00;**
**A61K 31/7004, A61K 2300/00;**
**A61K 31/7016, A61K 2300/00;**
**A61K 31/713, A61K 2300/00**

**Description**

The present invention generally relates to the field of skin care. More particularly, the invention relates to a skin care composition that comprises growth-enhancing compounds which promote the development of a healthy skin microbiome. These growth-enhancing compounds are metabolized by the microorganisms of the skin microbiome after their topical application to the skin, thereby supporting growth of the microorganisms. The growth-enhancing compounds and compositions comprising same can be used for treating or preventing skin diseases, for promoting the development of a healthy skin microbiome, for stabilizing a healthy skin microbiome, and for promoting growth of bacteria of the skin microbiome.

## BACKGROUND OF THE INVENTION

The human skin is colonized by millions of microorganisms, in particular bacteria and fungi. Most of these microorganisms are harmless or even beneficial to their host. The entirety of the microorganisms living on the human skin is referred to as the "skin microbiome". There is emerging evidence that the skin microbiome has a direct influence on human health. For example, bacteria of the skin microbiome contribute to health by secreting antimicrobial compounds that provide a barrier to infection of the body with pathogenic bacteria or fungi. In addition, the skin microbiome interacts with the immune system and effectively stimulates the immune system to combat pathogenic bacteria and fungi.

The human skin is a habitat that is generally unfavorable for microorganisms. The skin is salty, dry and continuously exposed to UV radiation. In addition, the availability of nutrients on the skin surface is limited to compounds that are released from dead skin cells of the upper epidermal layer. Due to these harsh conditions, the overall number of bacterial and fungal cells on skin is rather low, especially in dry areas such as the forearm, leg and lower back. Daily washing the skin with soap as well as the use of inappropiate cosmetic products provide additional antimicrobial effects which further reduce the overall number of bacterial and fungal cells on skin and may result in a disturbance of the skin microbiome.

In a healthy microbiome, there appears to be a balance of the resident microorganisms with respect to each other. A disturbance of said balance is thought to contribute to diseases or disorders, such as acne or eczema. In particular, if the number of bacteria from the resident flora, such as *Staphylococcus epidermidis,* becomes too low, there is a risk that the skin is colonized with pathogenic or potentially pathogenic bacteria, such as *Staphylococcus aureus.* A healthy and stable microbiome normally prevents the colonization of the skin with such pathogenic or potentially pathogenic bacteria. Accordingly, there is a need for means and methods that result in the maintainance of a healthy skin microbiome. In addition, there is a need for means and methods that could promote growth of the resident microorganisms in the microbiome.

## DESCRIPTION OF THE INVENTION

It has been found in the course of the present invention that it is possible to actively support a balanced microbiome by application of a skin care composition that comprises a high amount of compounds which can be metabolized by the microorganisms of the skin microbiome, thereby promoting their growth. In particular, it has been found that certain compounds, upon their topical application to the skin, can be used as carbon, nitrogen, or lipid source by the microorganisms of the skin microbiome. The topical application of such compounds therefore provides improved metabolic conditions for these microorganisms on the skin surface.

The growth-enhancing compounds disclosed herein are metabolized by the resident microorganisms, i.e. the microorganisms that normally occur as part of the human skin microbiome, much more effective compared to transient microorganisms which colonize the skin only temporarily, e.g. after touching contaminated objects with the hands. As a result, the compounds disclosed herein provide a growth advantage to resident microorganisms, such as *S. epidermidis,* over pathogenic microorganisms like *S. aureus.* In this way, these compounds assist in stabilizing and promoting growth of the resident microorganisms of the skin microbiome.

Accordingly, in a first aspect, the present invention relates to a skin care composition for promoting the skin microbiome, wherein said composition is formulated for topical application to the skin and comprises

> (a) a carbon source selected from the group consisting of glucose, fructose, saccharose, maltose, maltodextrin, pyruvate and/or lactate.

Apart from the carbon source, the skin care composition of the present invention further comprises at least one of following components:

> (b) a nitrogen source selected from the group of alanine, glycine, arginine and/or urea;

(c) caprylic/capric triglycerides as a lipid source.

Hence, the invention relates to a composition for promoting the skin microbiome which comprises a carbon source, which either is pyruvate, lactate or a combination of pyruvate and lactate, and at least one additional growth-enhancing compound. The additional growth-enhancing compound can be a nitrogen source selected from urea and arginine or caprylic/capric triglycerides as a lipid source. In a particularly preferred embodiment, the composition of the invention comprises all three types of components. Accordingly, in a preferred aspect, the invention relates to a skin care composition for promoting the skin microbiome, wherein said composition is formulated for topical application to the skin and comprises

(a) glucose, fructose, saccharose, maltose, maltodextrin, pyruvate and/or lactate as a carbon source,
(b) alanine, glycine, arginine and/or urea as a nitrogen source,
(c) caprylic/capric triglycerides as a lipid source.

In one preferred embodiment of the invention, the composition of the invention comprises pyruvate as a carbon source. As shown in the below examples, pyruvate is able to significantly increase the cell number of the resident bacteria when applied in the form of a skin care composition to the facial skin. As used herein, pyruvate refers to a salt or ester of 2-oxopropanoic acid. Preferably, the pyruvate in the composition is calcium or sodium pyruvate. Sodium pyruvate is used in some cosmetic compositions for stimulating collagen synthesis.

A preferred skin care composition of the invention comprises the following:

(a) pyruvate as a carbon source,
(b) urea as a nitrogen source, and
(c) as an optional component, caprylic/capric triglycerides as a lipid source.

It is preferred that the pyruvate is present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.7 to 2.0% (w/w), more preferably 0.8 to 1.5% (w/w), such as 1.0% (w/w). An amount of 0.05 to 1.0% (w/w) is particularly preferred. In some embodiments, the amount of pyruvate present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w).

Similarly, it is preferred that the urea is present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.7 to 2.0% (w/w), more preferably 0.8 to 1.5% (w/w), such as 1.0% (w/w). In some embodiments, the amount of urea present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w).

This composition may optionally include caprylic/capric triglycerides as a lipid source. The caprylic/capric triglycerides are preferably present in an amount of 0.1 to 6.0% (w/w), more preferably 0.2 to 6.0% (w/w), more preferably 0.3 to 5.5% (w/w), more preferably 0.4 to 5.0% (w/w), more preferably 0.5 to 4.5% (w/w), more preferably 0.6 to 4.0% (w/w), more preferably 0.7 to 3.5% (w/w), more preferably 0.8 to 3.0% (w/w), more preferably 0.9 to 2.5% (w/w), more preferably 1.0 to 3.0% (w/w), more preferably 1.5 to 2.5% (w/w), such as 2.0% (w/w). In some embodiments, the amount of caprylic/capric triglycerides present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), at least 3.0 (w/w), at least 4.0 (w/w), or at least 5.0 (w/w).

Another preferred skin care composition of the invention comprises the following:

(a) pyruvate as a carbon source,
(b) arginine a as a nitrogen source, and
(c) as an optional component, caprylic/capric triglycerides as a lipid source.

The pyruvate is preferably present in the composition in an amount as outlined above. The arginine is preferably present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.8 to 2.0% (w/w), more preferably 0.9 to 1.5% (w/w), such as 1.0% (w/w). In some embodiments, the amount of arginine present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w). This composition may optionally include caprylic/capric triglycerides as a lipid source. If present, the caprylic/capric triglycerides are preferably used in the amounts outlines above.

Another preferred skin care composition of the invention comprises the following:

(a) pyruvate as a carbon source,
(b) glycine a as a nitrogen source, and
(c) as an optional component, caprylic/capric triglycerides as a lipid source.

The pyruvate is preferably present in the composition in an amount as outlined above. The glycine is preferably present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.8 to 2.0% (w/w), more preferably 0.9 to 1.5% (w/w), such as 1.0% (w/w). In some embodiments, the amount of glycine present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w). This composition may optionally include caprylic/capric triglycerides as a lipid source. If present, the caprylic/capric triglycerides are preferably used in the amounts outlines above.

Another preferred skin care composition of the invention comprises the following:

(a) pyruvate as a carbon source,
(b) alanine a as a nitrogen source, and
(c) as an optional component, caprylic/capric triglycerides as a lipid source.

The pyruvate is preferably present in the composition in an amount as outlined above. The alanine is preferably present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.8 to 2.0% (w/w), more preferably 0.9 to 1.5% (w/w), such as 1.0% (w/w). In some embodiments, the amount of alanine present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w). This composition may optionally include caprylic/capric triglycerides as a lipid source. If present, the caprylic/capric triglycerides are preferably used in the amounts outlines above.

In another embodiment of the invention, the composition of the invention comprises lactate as a carbon source. Like pyruvate, lactate is able to increase the cell number of the resident bacteria when applied to the facial skin. As used herein, lactate refers to a salt or ester of lactic acid. Preferably, the lactate in the composition is calcium or sodium lactate. Sodium lactate is used as a humectant in some cosmetic compositions as well as for pH stabilization.

Preferred skin care compositions of the invention that comprise lactate as a growth-enhancing component, include:

A preferred skin care composition of the invention comprises the following:

(a) lactate as a carbon source,
(b) urea as a nitrogen source, and
(c) as an optional component, caprylic/capric triglycerides as a lipid source.

It is preferred that the lactate is present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.7 to 2.0% (w/w), more preferably 0.8 to 1.5% (w/w), such as 1.0% (w/w). In some embodiments, the amount of lactate present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w).

Similarly, it is preferred that the urea is present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.7 to 2.0% (w/w), more preferably 0.8 to 1.5% (w/w), such as 1.0% (w/w). In some embodiments, the amount of urea present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w).

This composition may optionally include caprylic/capric triglycerides as a lipid source. The caprylic/capric triglycerides are preferably present in an amount of 0.1 to 6.0% (w/w), more preferably 0.2 to 6.0% (w/w), more preferably 0.3 to 5.5% (w/w), more preferably 0.4 to 5.0% (w/w), more preferably 0.5 to 4.5% (w/w), more preferably 0.6 to 4.0% (w/w), more preferably 0.7 to 3.5% (w/w), more preferably 0.8 to 3.0% (w/w), more preferably 0.9 to 2.5% (w/w), more preferably 1.0 to 3.0% (w/w), more preferably 1.5 to 2.5% (w/w), such as 2.0% (w/w). In some embodiments, the amount of caprylic/capric triglycerides present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), at least 3.0 (w/w), at least 4.0 (w/w), or at least 5.0 (w/w).

Another preferred skin care composition of the invention comprises the following:

(a) lactate as a carbon source,
(b) arginine a as a nitrogen source, and
(c) as an optional component, caprylic/capric triglycerides as a lipid source.

The lactate is preferably present in the composition in an amount as outlined above. The arginine is preferably present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.8 to 2.0% (w/w), more preferably 0.9 to 1.5% (w/w), such as 1.0% (w/w). In some embodiments, the amount of arginine present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w). This composition may optionally include caprylic/capric triglycerides as a lipid source. If present, the caprylic/capric triglycerides are preferably used in the amounts outlines above.

Another preferred skin care composition of the invention comprises the following:

(a) lactate as a carbon source,
(b) glycine a as a nitrogen source, and
(c) as an optional component, caprylic/capric triglycerides as a lipid source.

The lactate is preferably present in the composition in an amount as outlined above. The glycine is preferably present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.8 to 2.0% (w/w), more preferably 0.9 to 1.5% (w/w), such as 1.0% (w/w). In some embodiments, the amount of glycine present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w). This composition may optionally include caprylic/capric triglycerides as a lipid source. If present, the caprylic/capric triglycerides are preferably used in the amounts outlines above.

Another preferred skin care composition of the invention comprises the following:

(a) lactate as a carbon source,
(b) alanine a as a nitrogen source, and
(c) as an optional component, caprylic/capric triglycerides as a lipid source.

The lactate is preferably present in the composition in an amount as outlined above. The alanine is preferably present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.8 to 2.0% (w/w), more preferably 0.9 to 1.5% (w/w), such as 1.0% (w/w). In some embodiments, the amount of alanine present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w). This composition may optionally include caprylic/capric triglycerides as a lipid source. If present, the caprylic/capric triglycerides are preferably used in the amounts outlines above.

Another preferred skin care composition of the invention comprises the following:

(a) glucose as a carbon source,
(b) urea as a nitrogen source, and
(c) as an optional component, caprylic/capric triglycerides as a lipid source.

It is preferred that the glucose is present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.7 to 2.0% (w/w), more preferably 0.8 to 1.5% (w/w), such as 1.0% (w/w). An amount of 0.05 to 1.0% (w/w) is particularly preferred. In some embodiments, the amount of glucose present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w).

Similarly, it is preferred that the urea is present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.7 to 2.0% (w/w), more preferably 0.8 to 1.5% (w/w), such as 1.0% (w/w). In some embodiments, the amount of urea present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w).

This composition may optionally include caprylic/capric triglycerides as a lipid source. The caprylic/capric triglycerides are preferably present in an amount of 0.1 to 6.0% (w/w), more preferably 0.2 to 6.0% (w/w), more preferably 0.3 to 5.5% (w/w), more preferably 0.4 to 5.0% (w/w), more preferably 0.5 to 4.5% (w/w), more preferably 0.6 to 4.0% (w/w), more preferably 0.7 to 3.5% (w/w), more preferably 0.8 to 3.0% (w/w), more preferably 0.9 to 2.5% (w/w), more preferably 1.0 to 3.0% (w/w), more preferably 1.5 to 2.5% (w/w), such as 2.0% (w/w). In some embodiments, the amount of caprylic/capric triglycerides present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), at least 3.0 (w/w), at least 4.0 (w/w), or at least 5.0 (w/w).

Another preferred skin care composition of the invention comprises the following:

(a) glucose as a carbon source,
(b) arginine a as a nitrogen source, and
(c) as an optional component, caprylic/capric triglycerides as a lipid source.

The glucose is preferably present in the composition in an amount as outlined above. The arginine is preferably present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.8 to 2.0% (w/w), more preferably 0.9 to 1.5% (w/w), such as 1.0% (w/w). In some embodiments, the amount of arginine present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w). This composition may optionally include caprylic/capric triglycerides as a lipid source. If present, the caprylic/capric triglycerides are preferably used in the amounts outlines above.

Another preferred skin care composition of the invention comprises the following:

(a) glucose as a carbon source,
(b) glycine a as a nitrogen source, and
(c) as an optional component, caprylic/capric triglycerides as a lipid source.

The glucose is preferably present in the composition in an amount as outlined above. The glycine is preferably present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.8 to 2.0% (w/w), more preferably 0.9 to 1.5% (w/w), such as 1.0% (w/w). In some embodiments, the amount of glycine present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w). This composition may optionally include caprylic/capric triglycerides as a lipid source. If present, the caprylic/capric triglycerides are preferably used in the amounts outlines above.

Another preferred skin care composition of the invention comprises the following:

(a) glucose as a carbon source,
(b) alanine a as a nitrogen source, and
(c) as an optional component, caprylic/capric triglycerides as a lipid source.

The glucose is preferably present in the composition in an amount as outlined above. The alanine is preferably present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.8 to 2.0% (w/w), more preferably 0.9 to 1.5% (w/w), such as 1.0% (w/w). In some embodiments, the amount of alanine present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w). This composition may optionally include caprylic/capric triglycerides as a lipid source. If present, the caprylic/capric triglycerides are preferably used in the amounts outlines above.

Another preferred skin care composition of the invention comprises the following:

(a) fructose as a carbon source,
(b) urea as a nitrogen source, and
(c) as an optional component, caprylic/capric triglycerides as a lipid source.

It is preferred that the fructose is present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.7 to 2.0% (w/w), more preferably 0.8 to 1.5% (w/w), such as 1.0% (w/w). An amount of 0.05 to 1.0% (w/w) is particularly preferred. In some embodiments, the amount of fructose present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w).

Similarly, it is preferred that the urea is present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.7 to 2.0% (w/w), more preferably 0.8 to 1.5% (w/w), such as 1.0% (w/w). In some embodiments, the amount of urea present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w).

This composition may optionally include caprylic/capric triglycerides as a lipid source. The caprylic/capric triglycerides are preferably present in an amount of 0.1 to 6.0% (w/w), more preferably 0.2 to 6.0% (w/w), more preferably 0.3 to 5.5% (w/w), more preferably 0.4 to 5.0% (w/w), more preferably 0.5 to 4.5% (w/w), more preferably 0.6 to 4.0% (w/w), more preferably 0.7 to 3.5% (w/w), more preferably 0.8 to 3.0% (w/w), more preferably 0.9 to 2.5% (w/w), more preferably 1.0 to 3.0% (w/w), more preferably 1.5 to 2.5% (w/w), such as 2.0% (w/w). In some embodiments, the amount of caprylic/capric triglycerides present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), at least 3.0 (w/w), at least 4.0 (w/w), or at least 5.0 (w/w).

Another preferred skin care composition of the invention comprises the following:

(a) fructose as a carbon source,
(b) arginine a as a nitrogen source, and
(c) as an optional component, caprylic/capric triglycerides as a lipid source.

The fructose is preferably present in the composition in an amount as outlined above. The arginine is preferably present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.8 to 2.0% (w/w), more preferably 0.9 to 1.5% (w/w), such as 1.0% (w/w). In some embodiments, the amount of arginine present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w). This composition may optionally include caprylic/capric triglycerides as a lipid source. If present, the caprylic/capric triglycerides are preferably used in the amounts outlines above.

Another preferred skin care composition of the invention comprises the following:

(a) fructose as a carbon source,
(b) glycine a as a nitrogen source, and
(c) as an optional component, caprylic/capric triglycerides as a lipid source.

The fructose is preferably present in the composition in an amount as outlined above. The glycine is preferably present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.8 to 2.0% (w/w), more preferably 0.9 to 1.5% (w/w), such as 1.0% (w/w). In some embodiments, the amount of glycine present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w). This composition may optionally include caprylic/capric triglycerides as a lipid source. If present, the caprylic/capric triglycerides are preferably used in the amounts outlines above.

Another preferred skin care composition of the invention comprises the following:

(a) fructose as a carbon source,
(b) alanine a as a nitrogen source, and
(c) as an optional component, caprylic/capric triglycerides as a lipid source.

The fructose is preferably present in the composition in an amount as outlined above. The alanine is preferably present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.8 to 2.0% (w/w), more preferably 0.9 to 1.5% (w/w), such as 1.0% (w/w). In some embodiments, the amount of alanine present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w). This composition may optionally include caprylic/capric triglycerides as a lipid source. If present, the caprylic/capric triglycerides are preferably used in the amounts outlines above.

Another preferred skin care composition of the invention comprises the following:

(a) maltose as a carbon source,
(b) urea as a nitrogen source, and
(c) as an optional component, caprylic/capric triglycerides as a lipid source.

It is preferred that the maltose is present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.7 to 2.0% (w/w), more preferably 0.8 to 1.5% (w/w), such as 1.0% (w/w). An amount of 0.05 to 1.0% (w/w) is particularly preferred. In some embodiments, the amount of maltose present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w).

Similarly, it is preferred that the urea is present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.7 to 2.0% (w/w), more preferably 0.8 to 1.5% (w/w), such as 1.0% (w/w). In some embodiments, the amount of urea present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w).

This composition may optionally include caprylic/capric triglycerides as a lipid source. The caprylic/capric triglycerides are preferably present in an amount of 0.1 to 6.0% (w/w), more preferably 0.2 to 6.0% (w/w), more preferably 0.3 to 5.5% (w/w), more preferably 0.4 to 5.0% (w/w), more preferably 0.5 to 4.5% (w/w), more preferably 0.6 to 4.0% (w/w), more preferably 0.7 to 3.5% (w/w), more preferably 0.8 to 3.0% (w/w), more preferably 0.9 to 2.5% (w/w), more preferably 1.0 to 3.0% (w/w), more preferably 1.5 to 2.5% (w/w), such as 2.0% (w/w). In some embodiments, the amount of caprylic/capric triglycerides present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), at least 3.0 (w/w), at least 4.0 (w/w), or at least 5.0 (w/w).

Another preferred skin care composition of the invention comprises the following:

(a) maltose as a carbon source,
(b) arginine a as a nitrogen source, and
(c) as an optional component, caprylic/capric triglycerides as a lipid source.

The maltose is preferably present in the composition in an amount as outlined above. The arginine is preferably present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.8 to 2.0% (w/w), more preferably 0.9 to 1.5% (w/w), such as 1.0% (w/w). In some embodiments, the amount of arginine present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w). This composition may optionally include caprylic/capric triglycerides as a lipid source. If present, the caprylic/capric triglycerides are preferably used in the amounts outlines above.

Another preferred skin care composition of the invention comprises the following:

(a) maltose as a carbon source,
(b) glycine a as a nitrogen source, and
(c) as an optional component, caprylic/capric triglycerides as a lipid source.

The maltose is preferably present in the composition in an amount as outlined above. The glycine is preferably present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.8 to 2.0% (w/w), more preferably 0.9 to 1.5% (w/w), such as 1.0% (w/w). In some embodiments, the amount of glycine present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w). This composition may optionally include caprylic/capric triglycerides as a lipid source. If present, the caprylic/capric triglycerides are preferably used in the amounts outlines above.

Another preferred skin care composition of the invention comprises the following:

(a) maltose as a carbon source,
(b) alanine a as a nitrogen source, and
(c) as an optional component, caprylic/capric triglycerides as a lipid source.

The maltose is preferably present in the composition in an amount as outlined above. The alanine is preferably present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.8 to 2.0% (w/w), more preferably 0.9 to 1.5% (w/w), such as 1.0% (w/w). In some embodiments, the amount of alanine present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w). This composition may optionally include caprylic/capric triglycerides as a lipid source. If present, the caprylic/capric triglycerides are preferably used in the amounts outlines above.

Another preferred skin care composition of the invention comprises the following:

(a) saccharose as a carbon source,
(b) urea as a nitrogen source, and
(c) as an optional component, caprylic/capric triglycerides as a lipid source.

It is preferred that the saccharose is present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.7 to 2.0% (w/w), more preferably 0.8 to 1.5% (w/w), such as 1.0% (w/w). An amount of 0.05 to 1.0% (w/w) is particularly preferred. In some embodiments, the amount of saccharose present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w).

Similarly, it is preferred that the urea is present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.7 to 2.0% (w/w), more preferably 0.8 to 1.5% (w/w), such as 1.0% (w/w). In some embodiments, the amount of urea present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w).

This composition may optionally include caprylic/capric triglycerides as a lipid source. The caprylic/capric triglycerides are preferably present in an amount of 0.1 to 6.0% (w/w), more preferably 0.2 to 6.0% (w/w), more preferably 0.3 to 5.5% (w/w), more preferably 0.4 to 5.0% (w/w), more preferably 0.5 to 4.5% (w/w), more preferably 0.6 to 4.0% (w/w), more preferably 0.7 to 3.5% (w/w), more preferably 0.8 to 3.0% (w/w), more preferably 0.9 to 2.5% (w/w), more preferably 1.0 to 3.0% (w/w), more preferably 1.5 to 2.5% (w/w), such as 2.0% (w/w). In some embodiments, the amount of caprylic/capric triglycerides present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), at least 3.0 (w/w), at least 4.0 (w/w), or at least 5.0 (w/w).

Another preferred skin care composition of the invention comprises the following:

(a) saccharose as a carbon source,
(b) arginine a as a nitrogen source, and
(c) as an optional component, caprylic/capric triglycerides as a lipid source.

The saccharose is preferably present in the composition in an amount as outlined above. The arginine is preferably present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.8 to 2.0% (w/w), more preferably 0.9 to 1.5% (w/w), such as 1.0% (w/w). In some embodiments, the amount of arginine present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w). This composition may optionally include caprylic/capric triglycerides as a lipid source. If present, the caprylic/capric triglycerides are preferably used in the amounts outlines above.

Another preferred skin care composition of the invention comprises the following:

(a) saccharose as a carbon source,
(b) glycine a as a nitrogen source, and
(c) as an optional component, caprylic/capric triglycerides as a lipid source.

The saccharose is preferably present in the composition in an amount as outlined above. The glycine is preferably present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.8 to 2.0% (w/w), more preferably 0.9 to 1.5% (w/w), such as 1.0% (w/w). In some embodiments, the amount of glycine present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w). This composition may optionally include caprylic/capric triglycerides as a lipid source. If present, the caprylic/capric triglycerides are preferably used in the amounts outlines above.

Another preferred skin care composition of the invention comprises the following:

(a) saccharose as a carbon source,
(b) alanine a as a nitrogen source, and
(c) as an optional component, caprylic/capric triglycerides as a lipid source.

The saccharose is preferably present in the composition in an amount as outlined above. The alanine is preferably present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.8 to 2.0% (w/w), more preferably 0.9 to 1.5% (w/w), such as 1.0% (w/w). In some embodiments, the amount of alanine present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w). This composition may optionally include caprylic/capric triglycerides as a lipid source. If present, the caprylic/capric triglycerides are preferably used in the amounts outlines above.

Another preferred skin care composition of the invention comprises the following:

(a) maltodextrin as a carbon source,
(b) urea as a nitrogen source, and
(c) as an optional component, caprylic/capric triglycerides as a lipid source.

It is preferred that the maltodextrin is present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.7 to 2.0% (w/w), more preferably 0.8 to 1.5% (w/w), such as 1.0% (w/w). An amount of 0.05 to 1.0% (w/w) is particularly preferred. In some embodiments, the amount of maltodextrin present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w).

Similarly, it is preferred that the urea is present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.7 to 2.0% (w/w), more preferably 0.8 to 1.5% (w/w), such as 1.0% (w/w). In some embodiments, the amount of urea present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w).

This composition may optionally include caprylic/capric triglycerides as a lipid source. The caprylic/capric triglycerides are preferably present in an amount of 0.1 to 6.0% (w/w), more preferably 0.2 to 6.0% (w/w), more preferably 0.3 to 5.5% (w/w), more preferably 0.4 to 5.0% (w/w), more preferably 0.5 to 4.5% (w/w), more preferably 0.6 to 4.0% (w/w), more preferably 0.7 to 3.5% (w/w), more preferably 0.8 to 3.0% (w/w), more preferably 0.9 to 2.5% (w/w), more preferably 1.0 to 3.0% (w/w), more preferably 1.5 to 2.5% (w/w), such as 2.0% (w/w). In some embodiments, the amount of caprylic/capric triglycerides present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), at least 3.0 (w/w), at least 4.0 (w/w), or at least 5.0 (w/w).

Another preferred skin care composition of the invention comprises the following:

(a) maltodextrin as a carbon source,
(b) arginine a as a nitrogen source, and
(c) as an optional component, caprylic/capric triglycerides as a lipid source.

The maltodextrin is preferably present in the composition in an amount as outlined above. The arginine is preferably present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.8 to 2.0% (w/w), more preferably 0.9 to 1.5% (w/w), such as 1.0% (w/w). In some embodiments, the amount of arginine present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w). This composition may optionally include caprylic/capric triglycerides as a lipid source. If present, the caprylic/capric triglycerides are preferably used in the amounts outlines above.

Another preferred skin care composition of the invention comprises the following:

(a) maltodextrin as a carbon source,
(b) glycine a as a nitrogen source, and
(c) as an optional component, caprylic/capric triglycerides as a lipid source.

The maltodextrin is preferably present in the composition in an amount as outlined above. The glycine is preferably present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.8 to 2.0% (w/w), more preferably 0.9 to 1.5% (w/w), such as 1.0% (w/w). In some embodiments, the amount of glycine present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w). This composition may optionally include caprylic/capric triglycerides as a lipid source. If present, the caprylic/capric triglycerides are preferably used in the amounts outlines above.

Another preferred skin care composition of the invention comprises the following:

(a) maltodextrin as a carbon source,
(b) alanine a as a nitrogen source, and
(c) as an optional component, caprylic/capric triglycerides as a lipid source.

The maltodextrin is preferably present in the composition in an amount as outlined above. The alanine is preferably present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.8 to 2.0% (w/w), more preferably 0.9 to 1.5% (w/w), such as 1.0% (w/w). In some embodiments, the amount of alanine present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w). This composition may optionally include caprylic/capric triglycerides as a lipid source. If present, the caprylic/capric triglycerides are preferably used in the amounts outlines above.

Particularly preferred compositions of the invention include, but are not limited to:
A skin care composition comprising:

(a) 0.05 to 5.0% (w/w) pyruvate as a carbon source,
(b) 0.05 to 5.0% (w/w) urea or alanine as a nitrogen source, and
(c) as an optional component, 0.05 to 6.0% (w/w) caprylic/capric triglycerides as a lipid source.

A skin care composition comprising:

(a) 0.05 to 5.0% (w/w) pyruvate as a carbon source,
(b) 0.05 to 5.0% (w/w) arginine or glycine as a nitrogen source, and
(c) as an optional component, 0.05 to 6.0% (w/w) caprylic/capric triglycerides as a lipid source.

A skin care composition comprising:

(a) 0.5 to 3.0% (w/w) pyruvate as a carbon source,
(b) 0.5 to 3.0% (w/w) urea or alanine as a nitrogen source, and
(c) as an optional component, 0.5 to 4.5% (w/w) caprylic/capric triglycerides as a lipid source.

A skin care composition comprising:

(a) 0.5 to 3.0% (w/w) pyruvate as a carbon source,
(b) 0.5 to 3.0% (w/w) arginine or glycine as a nitrogen source, and
(c) as an optional component, 0.5 to 4.5% (w/w) caprylic/capric triglycerides as a lipid source.

A skin care composition comprising:

(a) 1.0 to 1.5% (w/w) pyruvate as a carbon source,
(b) 1.0 to 1.5% (w/w) urea or alanine as a nitrogen source, and
(c) as an optional component, 1.5 to 2.5% (w/w) caprylic/capric triglycerides as a lipid source.

A skin care composition comprising:

(a) 1.0 to 1.5% (w/w) pyruvate as a carbon source,
(b) 1.0 to 1.5% (w/w) arginine or glycine as a nitrogen source, and
(c) as an optional component, 1.5 to 2.5% (w/w) caprylic/capric triglycerides as a lipid source.

A skin care composition comprising:

(a) 0.05 to 5.0% (w/w) lactate as a carbon source,
(b) 0.05 to 5.0% (w/w) urea or alanine as a nitrogen source, and
(c) as an optional component, 0.2 to 6.0% (w/w) caprylic/capric triglycerides as a lipid source.

A skin care composition comprising:

(a) 0.05 to 5.0% (w/w) lactate as a carbon source,
(b) 0.05 to 5.0% (w/w) arginine or glycine as a nitrogen source, and
(c) as an optional component, 0.2 to 6.0% (w/w) caprylic/capric triglycerides as a lipid source.

A skin care composition comprising:

(a) 0.5 to 3.0% (w/w) lactate as a carbon source,
(b) 0.5 to 3.0% (w/w) urea or alanine as a nitrogen source, and
(c) as an optional component, 0.5 to 4.5% (w/w) caprylic/capric triglycerides as a lipid source.

A skin care composition comprising:

(a) 0.5 to 3.0% (w/w) lactate as a carbon source,
(b) 0.5 to 3.0% (w/w) arginine or glycine as a nitrogen source, and
(c) as an optional component, 0.5 to 4.5% (w/w) caprylic/capric triglycerides as a lipid source.

A skin care composition comprising:

(a) 1.0 to 1.5% (w/w) lactate as a carbon source,
(b) 1.0 to 1.5% (w/w) urea or alanine as a nitrogen source, and
(c) as an optional component, 1.5 to 2.5% (w/w) caprylic/capric triglycerides as a lipid source.

A skin care composition comprising:

(a) 1.0 to 1.5% (w/w) lactate as a carbon source,
(b) 1.0 to 1.5% (w/w) arginine or glycine as a nitrogen source, and
(c) as an optional component, 1.5 to 2.5% (w/w) caprylic/capric triglycerides as a lipid source.

A skin care composition comprising:

(a) 0.05 to 5.0% (w/w) glucose as a carbon source,
(b) 0.05 to 5.0% (w/w) urea or alanine as a nitrogen source, and

(c) as an optional component, 0.05 to 6.0% (w/w) caprylic/capric triglycerides as a lipid source.

A skin care composition comprising:

(a) 0.05 to 5.0% (w/w) glucose as a carbon source,
(b) 0.05 to 5.0% (w/w) arginine or glycine as a nitrogen source, and
(c) as an optional component, 0.05 to 6.0% (w/w) caprylic/capric triglycerides as a lipid source.

A skin care composition comprising:

(a) 0.5 to 3.0% (w/w) glucose as a carbon source,
(b) 0.5 to 3.0% (w/w) urea or alanine as a nitrogen source, and
(c) as an optional component, 0.5 to 4.5% (w/w) caprylic/capric triglycerides as a lipid source.

A skin care composition comprising:

(a) 0.5 to 3.0% (w/w) glucose as a carbon source,
(b) 0.5 to 3.0% (w/w) arginine or glycine as a nitrogen source, and
(c) as an optional component, 0.5 to 4.5% (w/w) caprylic/capric triglycerides as a lipid source.

A skin care composition comprising:

(a) 1.0 to 1.5% (w/w) glucose as a carbon source,
(b) 1.0 to 1.5% (w/w) urea or alanine as a nitrogen source, and
(c) as an optional component, 1.5 to 2.5% (w/w) caprylic/capric triglycerides as a lipid source.

A skin care composition comprising:

(a) 1.0 to 1.5% (w/w) glucose as a carbon source,
(b) 1.0 to 1.5% (w/w) arginine or glycine as a nitrogen source, and
(c) as an optional component, 1.5 to 2.5% (w/w) caprylic/capric triglycerides as a lipid source.

A skin care composition comprising:

(a) 0.05 to 5.0% (w/w) fructose as a carbon source,
(b) 0.05 to 5.0% (w/w) urea or alanine as a nitrogen source, and
(c) as an optional component, 0.2 to 6.0% (w/w) caprylic/capric triglycerides as a lipid source.

A skin care composition comprising:

(a) 0.05 to 5.0% (w/w) fructose as a carbon source,
(b) 0.05 to 5.0% (w/w) arginine or glycine as a nitrogen source, and
(c) as an optional component, 0.2 to 6.0% (w/w) caprylic/capric triglycerides as a lipid source.

A skin care composition comprising:

(a) 0.5 to 3.0% (w/w) fructose as a carbon source,
(b) 0.5 to 3.0% (w/w) urea or alanine as a nitrogen source, and
(c) as an optional component, 0.5 to 4.5% (w/w) caprylic/capric triglycerides as a lipid source.

A skin care composition comprising:

(a) 0.5 to 3.0% (w/w) fructose as a carbon source,
(b) 0.5 to 3.0% (w/w) arginine or glycine as a nitrogen source, and
(c) as an optional component, 0.5 to 4.5% (w/w) caprylic/capric triglycerides as a lipid source.

A skin care composition comprising:

(a) 1.0 to 1.5% (w/w) fructose as a carbon source,
(b) 1.0 to 1.5% (w/w) urea or alanine as a nitrogen source, and
(c) as an optional component, 1.5 to 2.5% (w/w) caprylic/capric triglycerides as a lipid source.

A skin care composition comprising:

(a) 1.0 to 1.5% (w/w) fructose as a carbon source,
(b) 1.0 to 1.5% (w/w) arginine or glycine as a nitrogen source, and
(c) as an optional component, 1.5 to 2.5% (w/w) caprylic/capric triglycerides as a lipid source.

A skin care composition comprising:

(a) 0.05 to 5.0% (w/w) maltose as a carbon source,
(b) 0.05 to 5.0% (w/w) urea or alanine as a nitrogen source, and
(c) as an optional component, 0.05 to 6.0% (w/w) caprylic/capric triglycerides as a lipid source.

A skin care composition comprising:

(a) 0.05 to 5.0% (w/w) maltose as a carbon source,
(b) 0.05 to 5.0% (w/w) arginine or glycine as a nitrogen source, and
(c) as an optional component, 0.05 to 6.0% (w/w) caprylic/capric triglycerides as a lipid source.

A skin care composition comprising:

(a) 0.5 to 3.0% (w/w) maltose as a carbon source,
(b) 0.5 to 3.0% (w/w) urea or alanine as a nitrogen source, and
(c) as an optional component, 0.5 to 4.5% (w/w) caprylic/capric triglycerides as a lipid source.

A skin care composition comprising:

(a) 0.5 to 3.0% (w/w) maltose as a carbon source,
(b) 0.5 to 3.0% (w/w) arginine or glycine as a nitrogen source, and
(c) as an optional component, 0.5 to 4.5% (w/w) caprylic/capric triglycerides as a lipid source.

A skin care composition comprising:

(a) 1.0 to 1.5% (w/w) maltose as a carbon source,
(b) 1.0 to 1.5% (w/w) urea or alanine as a nitrogen source, and
(c) as an optional component, 1.5 to 2.5% (w/w) caprylic/capric triglycerides as a lipid source.

A skin care composition comprising:

(a) 1.0 to 1.5% (w/w) maltose as a carbon source,
(b) 1.0 to 1.5% (w/w) arginine or glycine as a nitrogen source, and
(c) as an optional component, 1.5 to 2.5% (w/w) caprylic/capric triglycerides as a lipid source.

A skin care composition comprising:

(a) 0.05 to 5.0% (w/w) saccharose as a carbon source,
(b) 0.05 to 5.0% (w/w) urea or alanine as a nitrogen source, and
(c) as an optional component, 0.2 to 6.0% (w/w) caprylic/capric triglycerides as a lipid source.

A skin care composition comprising:

(a) 0.05 to 5.0% (w/w) saccharose as a carbon source,
(b) 0.05 to 5.0% (w/w) arginine or glycine as a nitrogen source, and
(c) as an optional component, 0.2 to 6.0% (w/w) caprylic/capric triglycerides as a lipid source.

A skin care composition comprising:

(a) 0.5 to 3.0% (w/w) saccharose as a carbon source,
(b) 0.5 to 3.0% (w/w) urea or alanine as a nitrogen source, and
(c) as an optional component, 0.5 to 4.5% (w/w) caprylic/capric triglycerides as a lipid source.

A skin care composition comprising:

(a) 0.5 to 3.0% (w/w) saccharose as a carbon source,
(b) 0.5 to 3.0% (w/w) arginine or glycine as a nitrogen source, and
(c) as an optional component, 0.5 to 4.5% (w/w) caprylic/capric triglycerides as a lipid source.

A skin care composition comprising:

(a) 1.0 to 1.5% (w/w) saccharose as a carbon source,
(b) 1.0 to 1.5% (w/w) urea or alanine as a nitrogen source, and
(c) as an optional component, 1.5 to 2.5% (w/w) caprylic/capric triglycerides as a lipid source.

A skin care composition comprising:

(a) 1.0 to 1.5% (w/w) saccharose as a carbon source,
(b) 1.0 to 1.5% (w/w) arginine or glycine as a nitrogen source, and
(c) as an optional component, 1.5 to 2.5% (w/w) caprylic/capric triglycerides as a lipid source.

A skin care composition comprising:

(a) 0.05 to 5.0% (w/w) maltodextrin as a carbon source,
(b) 0.05 to 5.0% (w/w) urea or alanine as a nitrogen source, and
(c) as an optional component, 0.2 to 6.0% (w/w) caprylic/capric triglycerides as a lipid source.

A skin care composition comprising:

(a) 0.05 to 5.0% (w/w) maltodextrin as a carbon source,
(b) 0.05 to 5.0% (w/w) arginine or glycine as a nitrogen source, and
(c) as an optional component, 0.2 to 6.0% (w/w) caprylic/capric triglycerides as a lipid source.

A skin care composition comprising:

(a) 0.5 to 3.0% (w/w) maltodextrin as a carbon source,
(b) 0.5 to 3.0% (w/w) urea or alanine as a nitrogen source, and
(c) as an optional component, 0.5 to 4.5% (w/w) caprylic/capric triglycerides as a lipid source.

A skin care composition comprising:

(a) 0.5 to 3.0% (w/w) maltodextrin as a carbon source,
(b) 0.5 to 3.0% (w/w) arginine or glycine as a nitrogen source, and
(c) as an optional component, 0.5 to 4.5% (w/w) caprylic/capric triglycerides as a lipid source.

A skin care composition comprising:

(a) 1.0 to 1.5% (w/w) maltodextrin as a carbon source,
(b) 1.0 to 1.5% (w/w) urea or alanine as a nitrogen source, and
(c) as an optional component, 1.5 to 2.5% (w/w) caprylic/capric triglycerides as a lipid source.

A skin care composition comprising:

(a) 1.0 to 1.5% (w/w) maltodextrin as a carbon source,
(b) 1.0 to 1.5% (w/w) arginine or glycine as a nitrogen source, and

(c) as an optional component, 1.5 to 2.5% (w/w) caprylic/capric triglycerides as a lipid source.

Instead of a single amino acid like arginine or alanine, also mixtures of amino acids can be used according to the present invention, For example, mixtures comprising one or more of alanine, arginine, serine, threonine, histidine, glutamic acid and/or lysine can be used. Mixtures of alanine and arginine are particularly preferred. Another nitrogen source that can be used in the compositions of the invention, either alone or in combination with another nitrogen source, is betaine (2-trimethylammonioacetate). The betaine is preferably present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.8 to 2.0% (w/w), more preferably 0.9 to 1.5% (w/w), such as 1.0% (w/w). In some embodiments, the amount of betaine present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w).

In a preferred aspect of the invention, the skin care composition of the invention is formulated as a water-containing composition for topical application to the skin that has a particularly low osmolality. It has been found that it is possible to further support a healthy and stable microbiome by reducing osmolality. A high number of osmotically active solutes in the compositions leads to a high level of osmotic stress after application of the composition to the skin. After application to the skin, the water contained in the composition evaporates to a significant extent so that the remaining water on the skin surface comprises salts and other osmotically active components in highly concentrated form. These concentrated compounds are able to bind free water in the environment which is crucial for the bacterial metabolism and hence for viability of the bacteria. In addition, due to the hyperosmotic conditions on the skin surface, water from inside of the bacterial cells diffuses along the osmotic gradient to the outside. This severely impacts viability of the bacterial cells.

The osmolality of a solution quantifies the osmotically active particles or ions contained in a solution. In contrast to molarity, which determines the number of particles or ions in a volume of fluid, osmolality refers to the mass weight. It is normally expressed as mOsm/kg water or mmol/kg water. The actual osmolality of a composition can be determined in accordance with methods well-known in the art. For example, osmolality can be measured by using a freezing point depression osmometer or a vapour pressure depression osmometer. Since the freezing point of a water-based composition is depressed dependent on the number of osmotically active particles or ions contained, it is possible to measure osmolality by determining the freezing point of the composition.

Since measuring the actual osmolality is cumbersome, the osmolality of a composition is often determined in practice by calculation the "theoretical osmolality" based on the molar masses of the compounds contained in said composition and the amounts of the compounds in the composition. As used herein, the theoretical osmolality $b_{osm}$ indicates the molality of the osmotically active particles in a solution. It is calculated according to the formula:

$$b_{\mathrm{osm}} = \frac{n_{\mathrm{osm}}}{m_{\mathsf{Solvent}}}$$

wherein $n_{osm}$ indicates the amount of osmotically active particles in moles and $m_{Solvent}$ indicates the mass of the solvent in kg.

According to the invention, the skin care composition has a theoretical osmolality of 4000 mOsm/kg water or less. Preferably, the composition has a theoretical osmolality of 3900 mOsm/kg water or less, 3800 mOsm/kg water or less, 3700 mOsm/kg water or less, 3600 mOsm/kg water or less, 3500 mOsm/kg water or less, 3400 mOsm/kg water or less, 3300 mOsm/kg water or less, 3200 mOsm/kg water or less, 3100 mOsm/kg water or less, or 3000 mOsm/kg water or less. Stated differently, it is particularly preferred that skin care composition has a theoretical osmolality in the range between 3000-4000 mOsm/kg water, more preferably between 3100-3900 mOsm/kg water, between 3200-3800 mOsm/kg water, between 3300-3700 mOsm/kg water, or between 3400-3600 mOsm/kg water.

The composition of the invention preferably is a water-containing composition. The water content of the composition preferably is 30% (w/w) water or more. This means that the composition of the invention preferably comprises 30% (w/w) water or more, 35% (w/w) water or more, 40% (w/w) water or more, 45% (w/w) water or more, 50% (w/w) water or more, 55% (w/w) water or more, 60% (w/w) water or more, 65% (w/w) water or more, 70% (w/w) water or more, 75% (w/w) water or more, 80% (w/w) water or more, or 85% (w/w) water or more. It is particularly preferred that the composition of the invention comprises at least 70% (w/w) or at least 75% (w/w) water.

The skin care composition of the present invention is preferably formulated as a ready-to-use composition which is suitable for direct topical administration to the skin. Such a composition may be provided in different forms, including, but not limited to, in the form of a gel, lotion, fluid, cream, ointment, solution, a water-based emulsion, or the like. In a particularly preferred embodiment, the skin care composition of the present invention is formulated as a gel or lotion.

To provide for a low level of osmolality, the skin care compositions of the invention will use only reduced amounts of compounds which, upon their dissolution in water, are osmotically active. In some embodiments, certain osmotically active compounds like NaCl, KCl, $CaCl_2$ or $MgCl_2$ are completely omitted.

In one embodiment, the amount of NaCl in the composition which is to be administered to the skin is less than 1% (w/w), less than 0.9% (w/w), less than 0.8% (w/w), less than 0.7% (w/w), less than 0.6% (w/w), less than 0.5% (w/w), less than 0.4% (w/w), less than 0.3% (w/w), less than 0.2% (w/w), or less than 0.1% (w/w). In a particularly preferred embodiment, the composition does not comprise any NaCl.

In yet one embodiment, the amount of KCl in the composition which is to be administered to the skin is less than 1% (w/w), less than 0.9% (w/w), less than 0.8% (w/w), less than 0.7% (w/w), less than 0.6% (w/w), less than 0.5% (w/w), less than 0.4% (w/w), less than 0.3% (w/w), less than 0.2% (w/w), or less than 0.1% (w/w). In a particularly preferred embodiment, the composition does not comprise any KCl.

In one embodiment, the amount of $CaCl_2$ in the composition which is to be administered to the skin is less than 1% (w/w), less than 0.9% (w/w), less than 0.8% (w/w), less than 0.7% (w/w), less than 0.6% (w/w), less than 0.5% (w/w), less than 0.4% (w/w), less than 0.3% (w/w), less than 0.2% (w/w), or less than 0.1% (w/w). In a particularly preferred embodiment, the composition does not comprise any $CaCl_2$.

In one embodiment, the amount of $MgCl_2$ in the composition which is to be administered to the skin is less than 1% (w/w), less than 0.9% (w/w), less than 0.8% (w/w), less than 0.7% (w/w), less than 0.6% (w/w), less than 0.5% (w/w), less than 0.4% (w/w), less than 0.3% (w/w), less than 0.2% (w/w), or less than 0.1% (w/w). In a particularly preferred embodiment, the composition does not comprise any $MgCl_2$.

Another component that contributes to osmolality is glycerol. It is preferred according to the invention that the amount of glycerol in the composition which is to be administered to the skin is less than 10% (w/w), less than 9% (w/w), less than 8% (w/w), less than 7% (w/w), less than 6% (w/w), less than 5% (w/w), less than 4% (w/w), less than 3% (w/w), less than 2% (w/w), or less than 1% (w/w). In a particularly preferred embodiment, the composition does not comprise any glycerol.

In one embodiment, the skin care composition of the invention as defined elsewhere hereinabove comprises less than 0.5% (w/w) of NaCl and less than 5% (w/w) of glycerol. In one embodiment, the composition comprises less than 0.5% (w/w) of NaCl and no glycerol. In another embodiment, the composition comprises less than 5% (w/w) of glycerol and no NaCl.

In another embodiment, the skin care composition comprises less than 0.5% (w/w) of KCl and less than 5% (w/w) of glycerol. In one embodiment, the composition comprises less than 0.5% (w/w) of KCl and no glycerol. In another embodiment, the composition comprises less than 5% (w/w) of glycerol and no KCl.

In yet another embodiment, the skin care composition comprises less than 0.5% (w/w) of $CaCl_2$ and less than 5% (w/w) of glycerol. In one embodiment, the composition comprises less than 0.5% (w/w) of $CaCl_2$ and no glycerol. In another embodiment, the composition comprises less than 5% (w/w) of glycerol and no $CaCl_2$.

In yet another embodiment, the skin care composition comprises less than 0.5% (w/w) of $MgCl_2$ and less than 5% (w/w) of glycerol. In one embodiment, the composition comprises less than 0.5% (w/w) of $MgCl_2$ and no glycerol. In another embodiment, the composition comprises less than 5% (w/w) of glycerol and no $MgCl_2$.

A particularly preferred skin care composition of the invention therefore has an osmolality of 4000 mOsm/kg water or less and comprises

(a) glucose, fructose, saccharose, maltose, maltodextrin, pyruvate and/or lactate as a carbon source, preferably in an amount of 0.05 to 5.0% (w/w),
(b) at least 50% (w/w) water,
(c) less than 0.5% (w/w) of NaCl,
(d) less than 5% (w/w) of glycerol.

and at least one of the following:

(e) alanine, glycine, urea and/or arginine as a nitrogen source, preferably in an amount of 0.05 to 5.0% (w/w),
(f) caprylic/capric triglycerides as a lipid source preferably in an amount of 0.05 to 6.0% (w/w).

Another particularly preferred skin care composition of the invention therefore has an osmolality of 4000 mOsm/kg water or less and comprises

(a) glucose, fructose, saccharose, maltose, maltodextrin, pyruvate and/or lactate as a carbon source, preferably in an amount of 0.05 to 5.0% (w/w),
(b) at least 50% (w/w) water,

(c) less than 0.5% (w/w) of KCl,
(d) less than 5% (w/w) of glycerol.

and at least one of the following:

(e) alanine, glycine, urea and/or arginine as a nitrogen source, preferably in an amount of 0.05 to 5.0% (w/w),
(f) caprylic/capric triglycerides as a lipid source preferably in an amount of 0.05 to 6.0% (w/w).

Another particularly preferred skin care composition of the invention therefore has an osmolality of 4000 mOsm/kg water or less and comprises

(a) glucose, fructose, saccharose, maltose, maltodextrin, pyruvate and/or lactate as a carbon source, preferably in an amount of 0.05 to 5.0% (w/w),
(b) at least 50% (w/w) water,
(c) less than 0.5% (w/w) of $CaCl_2$,
(d) less than 5% (w/w) of glycerol.

and at least one of the following:

(e) alanine, glycine, urea and/or arginine as a nitrogen source, preferably in an amount of 0.05 to 5.0% (w/w),
(f) caprylic/capric triglycerides as a lipid source preferably in an amount of 0.05 to 6.0% (w/w).

Another particularly preferred skin care composition of the invention therefore has an osmolality of 4000 mOsm/kg water or less and comprises

(a) glucose, fructose, saccharose, maltose, maltodextrin, pyruvate and/or lactate as a carbon source, preferably in an amount of 0.05 to 5.0% (w/w),
(b) at least 50% (w/w) water,
(c) less than 0.5% (w/w) of $MgCl_2$,
(d) less than 5% (w/w) of glycerol.

and at least one of the following:

(e) alanine, glycine, urea and/or arginine as a nitrogen source, preferably in an amount of 0.05 to 5.0% (w/w),
(f) caprylic/capric triglycerides as a lipid source preferably in an amount of 0.05 to 6.0% (w/w).

A particularly preferred skin care composition of the invention therefore has an osmolality of 4000 mOsm/kg water or less and comprises

(a) 0.05 to 5.0% (w/w) glucose, fructose, saccharose, maltose, maltodextrin, pyruvate and/or lactate as a carbon source,
(b) 0.05 to 5.0% (w/w) urea as a nitrogen source,
(c) at least 50% (w/w) water,
(d) less than 0.5% (w/w) of NaCl,
(e) less than 5% (w/w) of glycerol, and
(f) as an optional component, 0.05 to 6.0% (w/w) caprylic/capric triglycerides as a lipid source.

A particularly preferred skin care composition of the invention therefore has an osmolality of 4000 mOsm/kg water or less and comprises

(a) 0.05 to 5.0% (w/w) glucose, fructose, saccharose, maltose, maltodextrin, pyruvate and/or lactate as a carbon source,
(b) 0.05 to 5.0% (w/w) arginine as a nitrogen source,
(c) at least 50% (w/w) water,
(d) less than 0.5% (w/w) of NaCl,
(e) less than 5% (w/w) of glycerol, and
(f) as an optional component, 0.05 to 6.0% (w/w) caprylic/capric triglycerides as a lipid source.

(a) 0.05 to 5.0% (w/w) glucose, fructose, saccharose, maltose, maltodextrin, pyruvate and/or lactate as a carbon source,
(b) 0.05 to 5.0% (w/w) glycine as a nitrogen source,
(c) at least 50% (w/w) water,
(d) less than 0.5% (w/w) of NaCl,
(e) less than 5% (w/w) of glycerol, and
(f) as an optional component, 0.05 to 6.0% (w/w) caprylic/capric triglycerides as a lipid source.

A particularly preferred skin care composition of the invention therefore has an osmolality of 4000 mOsm/kg water or less and comprises

(a) 0.05 to 5.0% (w/w) glucose, fructose, saccharose, maltose, maltodextrin, pyruvate and/or lactate as a carbon source,
(b) 0.05 to 5.0% (w/w) alanine as a nitrogen source,
(c) at least 50% (w/w) water,
(d) less than 0.5% (w/w) of NaCl,
(e) less than 5% (w/w) of glycerol, and
(f) as an optional component, 0.05 to 6.0% (w/w) caprylic/capric triglycerides as a lipid source.

A particularly preferred skin care composition of the invention therefore has an osmolality of 4000 mOsm/kg water or less and comprises

(a) 0.5 to 3.0% (w/w) glucose, fructose, saccharose, maltose, maltodextrin, pyruvate and/or lactate as a carbon source,
(b) 0.5 to 3.0% (w/w) urea as a nitrogen source,
(c) at least 50% (w/w) water,
(d) less than 0.5% (w/w) of NaCl,
(e) less than 5% (w/w) of glycerol, and
(f) as an optional component, 0.5 to 4.5% (w/w) caprylic/capric triglycerides as a lipid source.

A particularly preferred skin care composition of the invention therefore has an osmolality of 4000 mOsm/kg water or less and comprises

(a) 0.5 to 3.0% (w/w) glucose, fructose, saccharose, maltose, maltodextrin, pyruvate and/or lactate as a carbon source,
(b) 0.5 to 3.0% (w/w) arginine as a nitrogen source,
(c) at least 50% (w/w) water,
(d) less than 0.5% (w/w) of NaCl,
(e) less than 5% (w/w) of glycerol, and
(f) as an optional component, 0.5 to 4.5% (w/w) caprylic/capric triglycerides as a lipid source.

A particularly preferred skin care composition of the invention therefore has an osmolality of 4000 mOsm/kg water or less and comprises

(a) 0.5 to 3.0% (w/w) glucose, fructose, saccharose, maltose, maltodextrin, pyruvate and/or lactate as a carbon source,
(b) 0.5 to 3.0% (w/w) glycine as a nitrogen source,
(c) at least 50% (w/w) water,
(d) less than 0.5% (w/w) of NaCl,
(e) less than 5% (w/w) of glycerol, and
(f) as an optional component, 0.5 to 4.5% (w/w) caprylic/capric triglycerides as a lipid source.

A particularly preferred skin care composition of the invention therefore has an osmolality of 4000 mOsm/kg water or less and comprises

(a) 0.5 to 3.0% (w/w) glucose, fructose, saccharose, maltose, maltodextrin, pyruvate and/or lactate as a carbon source,
(b) 0.5 to 3.0% (w/w) alanine as a nitrogen source,

(c) at least 50% (w/w) water,
(d) less than 0.5% (w/w) of NaCl,
(e) less than 5% (w/w) of glycerol, and
(f) as an optional component, 0.5 to 4.5% (w/w) caprylic/capric triglycerides as a lipid source.

A particularly preferred skin care composition of the invention therefore has an osmolality of 4000 mOsm/kg water or less and comprises

(a) glucose, fructose, saccharose, maltose, maltodextrin, pyruvate and/or lactate as a carbon source, preferably in an amount of 0.05 to 5.0% (w/w),
(b) at least 50% (w/w) water,
(c) no NaCl,
(d) no glycerol.

and at least one of the following:

(e) alanine, glycine, urea and/or arginine as a nitrogen source, preferably in an amount of 0.05 to 5.0% (w/w),
(f) caprylic/capric triglycerides as a lipid source preferably in an amount of 0.05 to 6.0% (w/w).

Another particularly preferred skin care composition of the invention therefore has an osmolality of 4000 mOsm/kg water or less and comprises

(a) glucose, fructose, saccharose, maltose, maltodextrin, pyruvate and/or lactate as a carbon source, preferably in an amount of 0.05 to 5.0% (w/w),
(b) at least 50% (w/w) water,
(c) no NaCl,
(d) no glycerol,

and at least one of the following:

(e) alanine, glycine, urea and/or arginine as a nitrogen source, preferably in an amount of 0.05 to 5.0% (w/w),
(f) caprylic/capric triglycerides as a lipid source preferably in an amount of 0.05 to 6.0% (w/w).

Another particularly preferred skin care composition of the invention therefore has an osmolality of 4000 mOsm/kg water or less and comprises

(a) glucose, fructose, saccharose, maltose, maltodextrin, pyruvate and/or lactate as a carbon source, preferably in an amount of 0.05 to 5.0% (w/w),
(b) at least 50% (w/w) water,
(c) no NaCl,
(d) no glycerol,

and at least one of the following:

(e) alanine, glycine, urea and/or arginine as a nitrogen source, preferably in an amount of 0.05 to 5.0% (w/w),
(f) caprylic/capric triglycerides as a lipid source preferably in an amount of 0.05 to 6.0% (w/w).

Another particularly preferred skin care composition of the invention therefore has an osmolality of 4000 mOsm/kg water or less and comprises

(a) glucose, fructose, saccharose, maltose, maltodextrin, pyruvate and/or lactate as a carbon source, preferably in an amount of 0.05 to 5.0% (w/w),
(b) at least 50% (w/w) water,
(c) no NaCl,
(d) no glycerol,

and at least one of the following:

(e) alanine, glycine, urea and/or arginine as a nitrogen source, preferably in an amount of 0.05 to 5.0% (w/w),
(f) caprylic/capric triglycerides as a lipid source preferably in an amount of 0.05 to 6.0% (w/w).

A particularly preferred skin care composition of the invention therefore has an osmolality of 4000 mOsm/kg water or less and comprises

(a) 0.05 to 5.0% (w/w) glucose, fructose, saccharose, maltose, maltodextrin, pyruvate and/or lactate as a carbon source,
(b) 0.05 to 5.0% (w/w) urea as a nitrogen source,
(c) at least 50% (w/w) water,
(d) no NaCl,
(e) no glycerol,
(f) as an optional component, 0.05 to 6.0% (w/w) caprylic/capric triglycerides as a lipid source.

A particularly preferred skin care composition of the invention therefore has an osmolality of 4000 mOsm/kg water or less and comprises

(a) 0.05 to 5.0% (w/w) glucose, fructose, saccharose, maltose, maltodextrin, pyruvate and/or lactate as a carbon source,
(b) 0.05 to 5.0% (w/w) arginine as a nitrogen source,
(c) at least 50% (w/w) water,
(d) no NaCl,
(e) no glycerol,
(f) as an optional component, 0.05 to 6.0% (w/w) caprylic/capric triglycerides as a lipid source.

(a) 0.05 to 5.0% (w/w) glucose, fructose, saccharose, maltose, maltodextrin, pyruvate and/or lactate as a carbon source,
(b) 0.05 to 5.0% (w/w) glycine as a nitrogen source,
(c) at least 50% (w/w) water,
(d) no NaCl,
(e) no glycerol,
(f) as an optional component, 0.05 to 6.0% (w/w) caprylic/capric triglycerides as a lipid source.

A particularly preferred skin care composition of the invention therefore has an osmolality of 4000 mOsm/kg water or less and comprises

(a) 0.05 to 5.0% (w/w) glucose, fructose, saccharose, maltose, maltodextrin, pyruvate and/or lactate as a carbon source,
(b) 0.05 to 5.0% (w/w) alanine as a nitrogen source,
(c) at least 50% (w/w) water,
(d) no NaCl,
(e) no glycerol,
(f) as an optional component, 0.05 to 6.0% (w/w) caprylic/capric triglycerides as a lipid source.

A particularly preferred skin care composition of the invention therefore has an osmolality of 4000 mOsm/kg water or less and comprises

(a) 0.5 to 3.0% (w/w) glucose, fructose, saccharose, maltose, maltodextrin, pyruvate and/or lactate as a carbon source,
(b) 0.5 to 3.0% (w/w) urea as a nitrogen source,
(c) at least 50% (w/w) water,
(d) no NaCl,
(f) no glycerol,
(f) as an optional component, 0.5 to 4.5% (w/w) caprylic/capric triglycerides as a lipid source.

A particularly preferred skin care composition of the invention therefore has an osmolality of 4000 mOsm/kg water or less and comprises

(a) 0.5 to 3.0% (w/w) glucose, fructose, saccharose, maltose, maltodextrin, pyruvate and/or lactate as a carbon source,
(b) 0.5 to 3.0% (w/w) arginine as a nitrogen source,
(c) at least 50% (w/w) water,
(d) no NaCl,
(e) no glycerol,
(f) as an optional component, 0.5 to 4.5% (w/w) caprylic/capric triglycerides as a lipid source.

A particularly preferred skin care composition of the invention therefore has an osmolality of 4000 mOsm/kg water or less and comprises

(a) 0.5 to 3.0% (w/w) glucose, fructose, saccharose, maltose, maltodextrin, pyruvate and/or lactate as a carbon source,
(b) 0.5 to 3.0% (w/w) glycine as a nitrogen source,
(c) at least 50% (w/w) water,
(d) no NaCl,
(e) no glycerol,
(f) as an optional component, 0.5 to 4.5% (w/w) caprylic/capric triglycerides as a lipid source.

A particularly preferred skin care composition of the invention therefore has an osmolality of 4000 mOsm/kg water or less and comprises

(a) 0.5 to 3.0% (w/w) glucose, fructose, saccharose, maltose, maltodextrin, pyruvate and/or lactate as a carbon source,
(b) 0.5 to 3.0% (w/w) alanine as a nitrogen source,
(c) at least 50% (w/w) water,
(d) no NaCl,
(e) no glycerol,
(f) as an optional component, 0.5 to 4.5% (w/w) caprylic/capric triglycerides as a lipid source.

The skin care composition of the present invention can further comprise additional compounds which are normally present in topical skin compositions. For example, the skin care composition of the present invention can further comprise at least one compound selected from the group consisting of perfumes, emollients, pigments, thickener, fillers, colorants, antioxidants, surfactants, lubricants, stabilizers, preservatives, solubilizers, emulsifiers and combinations of any of these.

The skin care compositions described herein are particularly useful for promoting and maintaining a healthy skin microbiome. The compositions preferably increase the overall number of microorganisms, more preferably the overall number of bacteria, in a skin areal to which the skin care compositions are applied. It is particularly preferred that the overall number of microorganisms, more preferably the overall number of bacteria, is increased after daily topical application of the composition over a period of 2-4 weeks by at least 2.5%, by at least 5%, by at least 7.5%, by at least 10%, by at least 12.5%, or by at least 15% relative to an untreated areal of the part of the body. It is particularly preferred that the number of bacteria of the species *S. epidermidis* is increased after daily topical application of the composition over a period of 2-4 weeks. Preferably, the number of *S. epidermidis* is increased by at least 2.5%, by at least 5%, by at least 7.5%, by at least 10%, by at least 12.5%, or by at least 15% relative to an untreated areal of the part of the body.

The compositions of the invention are useful for therapeutic purposes, in particular for preventing or treating skin diseases like acne. Hence, in another aspect the invention provides a skin care composition as defined hereinabove for use in a method of treating a skin disease, preferably acne. In one embodiment, the skin care composition is used for preventing the formation of acne. In another embodiment, the skin care composition is used for treating an acute state of acne. In yet another preferred embodiment, the skin care composition is used for preventing the reoccurrence of acne in a subject who has received a standard acne treatment. It is particularly preferred that the subject is a human.

The invention also provides methods for treating the skin of a subject by administering a skin care composition as described hereinabove. In one aspect, a method of treating a skin disease, preferably acne, is provided, said method comprising the topical administration of a skin care composition as described hereinabove.

Apart from therapeutic uses, the compositions of the invention can also be used for non-therapeutic, cosmetic purposes, e.g. for promoting the development of or stabilizing a healthy skin microbiome, for improving the appearance of the skin of a subject, for maintaining healthy skin, or for promoting bacterial growth of the skin microbiome. Accordingly, the present invention also relates to the use, preferably the non-therapeutic use, of a composition comprising

(a) a carbon source selected from the group consisting of pyruvate and lactate, and/or

(b) a nitrogen source selected from the group consisting of urea and arginine; and/or

(c) caprylic/capric triglycerides

for promoting the development of or stabilizing a healthy skin microbiome or for promoting bacterial growth of the skin microbiome. In one embodiment, pyruvate is used as a carbon source for promoting the development of or stabilizing a healthy skin microbiome or for promoting bacterial growth of the skin microbiome. The pyruvate can be, for example, calcium or sodium pyruvate. More preferably, the pyruvate is used in combination with urea and/or arginine as a nitrogen source for promoting the development of or stabilizing a healthy skin microbiome or for promoting bacterial growth of the skin microbiome. Most preferably, the pyruvate is used in combination with urea and/or arginine as a nitrogen source and caprylic/capric triglycerides as a lipid source for promoting the development of or stabilizing a healthy skin microbiome or for promoting bacterial growth of the skin microbiome.

In another embodiment, lactate is used as a carbon source for promoting the development of or stabilizing a healthy skin microbiome or for promoting bacterial growth of the skin microbiome. The lactate can be, for example, calcium or sodium lactate. More preferably, the lactate is used in combination with urea and/or arginine as a nitrogen source for promoting the development of or stabilizing a healthy skin microbiome or for promoting bacterial growth of the skin microbiome. Most preferably, the lactate is used in combination with urea and/or arginine as a nitrogen source and caprylic/capric triglycerides as a lipid source for promoting the development of or stabilizing a healthy skin microbiome or for promoting bacterial growth of the skin microbiome.

## BRIEF DESCRIPTION OF THE FIGURE

Figure 1 shows the results from *in vitro* studies for promoting bacterial cell growth. It can be seen that the addition of pyruvate or lactate as a carbon source, either alone or in combination with a nitrogen source and/or a lipid source, induced a significant growth of S. *epidermidis* within 24 hours.

Figure 2 shows further results from *in vitro* studies for promoting bacterial cell growth. It can be seen that the addition of pyruvate, either alone or in combination with arginine, resulted in a significant growth of *S. epidermidis* within 24 hours.

Figure 3 shows the results from the analysis of skin hydration and cell count in a volunteer study. More specifically, Figure 3A shows the results from measuring skin hydration with a corneometer after application of compositions of the invention to the thigh skin for four weeks in an *in vivo* study. Figure 3B shows the results of the cell count measurements from the same study. It can be seen that the compositions of the invention led to significant skin hydration both after 2 and 4 weeks. Also, the compositions led to an increase in the cell number in the microbiome.

Figure 4 shows the results from 16S amplicon sequencing after applying the composition of the invention in vivo. No changes in the alpha and beta diversity were observed between the treatment group and the control group.

## EXAMPLES

The present invention is further illustrated by the following examples, which in no way should be construed as limiting. The entire contents of all of the references (including literature references, issued patents, published patent applications, and co pending patent applications) cited throughout this application are hereby expressly incorporated by reference.

Example 1: In vitro growth assays

Potential growth-enhancing compounds that are effective in the stabilization of the microbiome were tested by the following *in vitro* assay. Overnight cultures of *S. epidermidis* is inoculated in 20 ml CASO medium and incubated at 37°C. The culture was then diluted and adjusted to an OD600 of 0.25. From this suspension, the starting culture is prepared with a concentration of $5 \times 10^5$ CFU/ml by a 1:200 dilution in CASO deficiency medium. Subsequently, solutions containing the different active ingredients were mixed with the start cultures and incubated in a thermal mixer at 37°C by shaking. Samples are taken at the respective time points for testing, diluted with NaCl and plated out on CASO plates using the spiral plater. The plates are incubated for 24h at 37°C and then counted.

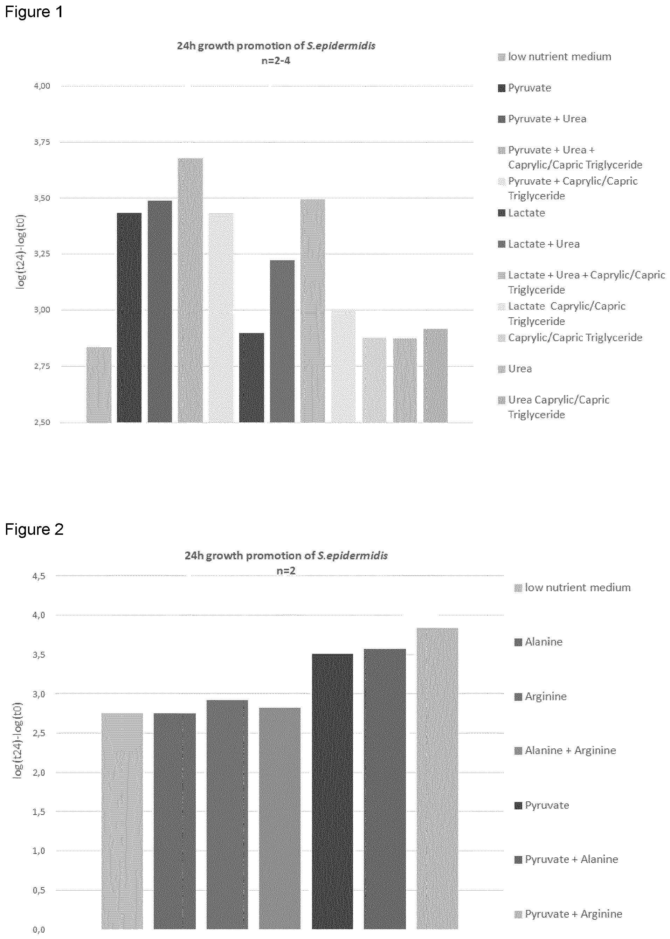

Results: The results can be shown in Figures 1 and 2. It can be taken from these figures that the addition of pyruvate or lactate to the low nutrient medium significantly promotes the growth of *S. epidermidis* within the 24 hours test period. The growth of the organism can even be further promoted by adding urea, argine or caprylic/capric triglycerides to the pyruvate or lactate.

Example 2: *In vivo* study

An in vivo study with 31 volunteers was conducted to examine whether the growth-enhancing compounds that were found effective in the *in vitro* test can also promote cell growth of the microorganisms in the skin microbiome *in vivo* when applied to the skin in the form of a lotion. For this purpose, a carrier lotion was prepared to which the test compounds, i.e. the compounds identified as growth-enhancing in the in *vitro* assays of Example 1, were added.

The composition of the carrier lotion is described in the following table.

Table 1: Composition of the carrier lotion

| INCI | wt. % |
|---|---|
| ethylhexyl salicylate | 5.00 |
| butyrospermum parkii butter | 4.50 |
| polysorbate 60 | 2.50 |
| glycerin | 1.50 |
| 4-methylbenzylidene camphor | 1.00 |
| glyceryl stearate | 1.00 |
| PEG-100 stearate | 1.00 |
| C12-15 alkyl benzoate | 1.00 |
| panthenol | 1.00 |
| phenoxyethanol | 0.70 |
| butyl methoxydibenzoylmethane | 0.60 |
| oryza sativa starch | 0.50 |
| hydroxyacetophenone | 0.40 |
| ethylhexylglycerin | 0.30 |
| tocopheryl acetate | 0.10 |
| tetrasodium glutamate diacetate | 0.10 |
| aqua | ad 100% |

The test formulations were prepared by mixing the compounds to be tested into the carrier lotion. The test formulations were composed as follows:

Formulation 20: Carrier lotion + 1% urea, 0.3% pyruvate, 1% caprylic/capric triglycerides
Formulation 30: Carrier lotion + 1% urea, 1% sodium Lactate
Formulation 40: Carrier lotion + 1% urea, 1% sodium Lactate, 1% caprylic/capric triglycerides

For preconditioning, the study participants ceased to cream the thighs or wash them with shower gel or soap 1 week before the start of the study. 4 test areas were marked on the thighs of each participant (01 untreated control / test formulation 20 / test formulation 30 / test formulation 40). The above test formulations were applied two times a day within the marked areas of the thighs in the morning and evening by the test person at home. After 2 weeks and after 4 weeks, the hydration of the skin in the test areas was measured and samples of the microbiome were collected by swabbing on the thighs.

Example 3: Test area and sample analysis

The microbiome samples obtained from the *in vivo* study of Example 2 were analyzed by microbial colony count and bacterial diversity via 16S rRNA amplicon sequencing. In addition, the tested skin areal was analyzed by measuring skin hydration.

For measuring skin hydration, the skin areas of the study participants were measured with a Corneometer CM 825 (Courage & Khazaka, Cologne, Germany) using 5 repeated measurements. Skin hydration was expressed as permittivity in arbitrary units [a. u.], wherein an increase indicates an improved skin hydration.

For measuring the microbial colony count, the test site was rinsed with swabs (FLOQSwabs, Hain Lifescience GmbH, Nehren, Germany) by 3 x 10 lanes using 2 ml rinsing buffer (12,49 g/L $Na_2HPO$ ; 0,63 g/L $KH_2PO_4$ in molecular grade $H_2O$) in 15 ml Falcon tubes. The sample material was then plated within 60 minutes after collection on Columbia-Sheep-Blood-Tween (COST) agar plates and subsequently incubated. Specifically, 10 μl of the rinsing buffer was diluted in 990 μl 0.9% NaCl solution (1:100), and 100 μl of this dilution as well as 100 μl of the undiluted rinse was placed on COST plates with the spiral plater and subsequently incubated at 37°C for 24 h. After incubation, the bacterial colonies on agar plates were counted using the Countermat Flash.

For the molecular 16S rRNA analysis, the microorganisms obtained by rinsing the test site were subjected to the DNA extraction, 16S rRNA amplicon sequencing and subsequent bio-informatics analysis. The aim of this analysis was to detect changes in the bacterial diversity compared to baseline which would indicate an influence of the test formulation on the on the composition of the skin microbiome.

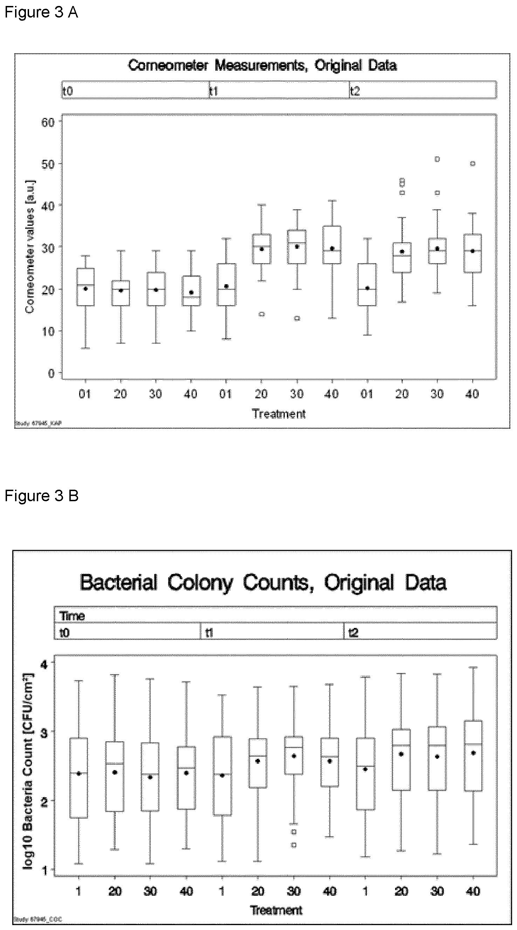

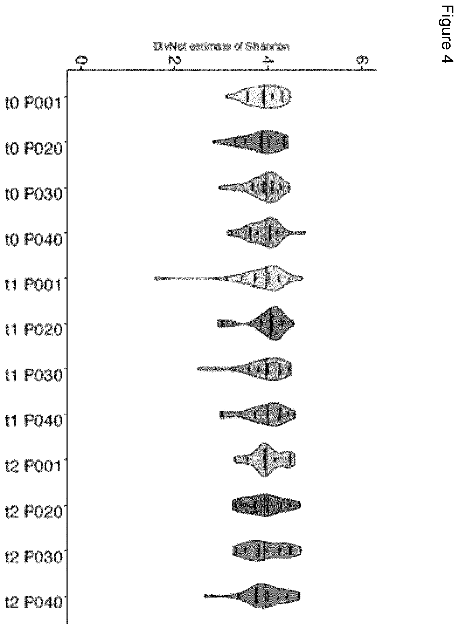

Results: The results of skin hydration measurements are depicted in Figure 3. As can be seen in Figure 3A, the test formulations provide for a significant skin hydration both after 2 and 4 weeks. In addition, as shown in Figure 3B, the test formulations led to significant increase in the cell count. Figure 4 shows the results from 16S amplicon sequencing. No evident changes in the alpha and beta diversity were observed between the treatment group and the control group. Accordingly, the test formulations stabilize the skin microbiome and promote growth of the bacteria in the skin microbiome without changing the composition of the microbiome.

Example 4: In vitro growth assays (OD)

Potential growth-enhancing compounds that are effective in the stabilization of the microbiome were tested by the following *in vitro* assay. Overnight cultures of S. *epidermidis* were inoculated in 20 ml CASO medium and incubated at 37°C. The culture was then diluted and adjusted to an OD600 of 0.25. From this suspension, the starting culture is prepared with a concentration of 5 x $10^5$ CFU/ml by a 1:200 dilution in CASO deficiency medium. Subsequently, solutions containing the different nitrogen, carbon, and lipid sources were mixed with the start cultures and 200 μl/well were pipetted into a 96 well flat transparent plate, at least in triplets. The plate was placed in a Tecan Reader at 37°C and Absorption was measured at 600 nm every hour over a period of 24-30h.

Results: The results of the in vitro growth tests is depicted in the below tables 2 and 3. It can be seen in Table 2 that the carbon sources glucose, fructose, saccharose, maltose, maltodextrin, pyruvate and lactate support the growth of S. *epidermidis.* Further, Table 3 shows that also the combination of the carbon source pyruvate with nitrogen sources like urea, alanine (Ala) and arginine (Arg) supports growth. In addition, a combination of the carbon source pyruvate or lactate with caprylic/capric triglycerides (FettK12) supports growth.

| Substance | Growth S. epidermidis | | |
|---|---|---|---|
| | 0,10% | 1,00% | 5,00% |
| Glucose | ++ | ++ | ++ |
| Pyruvate | + | ++ | ++ |
| Fructose | + | ++ | ++ |
| Maltose | + | ++ | ++ |
| Sucrose | + | ++ | ++ |
| Maltodextrin | + | ++ | ++ |
| Mannitol | X | X | X |
| Sorbitol | X | X | X |
| Xylitol | X | X | X |
| Lactate | + | ++ | X |

Table 2: Results from *in vitro* growth assays

| Substance | Growth *S. epidermidis* | | |
| --- | --- | --- | --- |
| | 0.1% | 1.00% | 5.00% |
| Pyruvate | + | ++ | + |
| Arg +Ala | | X | |
| Arg + Pyr | | ++ | |
| Ala + Pyr | | ++ | |
| Ala + Arg + Pyr | | ++ | |
| Pyruvate | | ++ | |
| Pyruvate + Urea | | ++ | |
| Pyruvate + Urea + Fettk12 | | ++ | |
| Pyruvate + Fettk12 | | ++ | |
| Lactate | | ++ | |
| Lactate+ Urea | | ++ | |
| Lactate+ Urea + Fettk12 | | ++ | |
| Lactate+ Fettk12 | | ++ | |
| Urea | | X | |
| Urea + Fettk12 | | + | |
| Fettk12 | | + | |

Table 3: Results from *in vitro* growth assays

**Claims**

1. Skin care composition for promoting the skin microbiome, wherein said composition is formulated for topical application to the skin and comprises

   (a) a carbon source selected from the group consisting of glucose, fructose, saccharose, maltose, maltodextrin, pyruvate and/or lactate, preferably in an amount of 0.05 to 5.0% (w/w),

   and at least one of the following:

   (b) a nitrogen source selected from the group of alanine, glycine, arginine and/or urea, preferably in an amount of 0.05 to 5.0% (w/w),
   (c) caprylic/capric triglycerides as a lipid source preferably in an amount of 0.05 to 6.0% (w/w).

2. Skin care composition for promoting the skin microbiome of claim 1, wherein said carbon source is glucose, preferably in an amount of 0.5 to 3.0% (w/w).

3. Skin care composition for promoting the skin microbiome of claim 1, wherein said carbon source is fructose, preferably in an amount of 0.5 to 3.0% (w/w).

4. Skin care composition for promoting the skin microbiome according to any of claims 1-3, wherein said nitrogen source is glycine, preferably in an amount of 0.5 to 3.0% (w/w).

5. Skin care composition for promoting the skin microbiome according to any of claims 1-3, wherein said nitrogen source is alanine, preferably in an amount of 0.1 to 3.0% (w/w).

6. Skin care composition for promoting the skin microbiome according to any of claims 1-5, comprising

   (a) a carbon source selected from the group consisting of glucose, fructose, saccharose, maltose, maltodextrin, pyruvate and/or lactate;
   (b) a nitrogen source selected from the group consisting of alanine, glycine, arginine and/or urea
   (c) caprylic/capric triglycerides.

7. Skin care composition for promoting the skin microbiome according to any of claims 1-6, comprising

   (a) 0.5 to 3.0% (w/w) of a carbon source selected from the group consisting of glucose, fructose, saccharose, maltose, maltodextrin, pyruvate and/or lactate;
   (b) 0.1 to 3.0% (w/w) a nitrogen source selected from the group consisting of alanine, glycine, arginine and/or urea;
   (c) 0.5 to 4.5% (w/w) caprylic/capric triglycerides.

8. Skin care composition for promoting the skin microbiome according to any of claims 1-7, comprising

   (a) glucose as a carbon source;
   (b) alanine as a nitrogen source;
   (c) optionally, caprylic/capric triglycerides.

9. Skin care composition for promoting the skin microbiome according to claim 8, comprising

   (a) 0.5 to 3.0% (w/w) glucose as a carbon source;
   (b) 0.1 to 3.0% (w/w) alanine as a nitrogen source;
   (c) 0.5 to 4.5% (w/w) caprylic/capric triglycerides.

10. Skin care composition for promoting the skin microbiome according to any of claims 1-9, further comprising at least one compound selected from the group consisting of a perfume, an emollient, a pigment, a thickener, a filler, a colorant, an antioxidant, a surfactant, a lubricant, a stabilizer, a preservative, a solubilizer, an emulsifier or a combination of any of these.

11. Skin care composition for promoting the skin microbiome according to any of claims 1-10 for use in a method of treating a skin disease, preferably acne.

12. Non-therapeutic use of a composition comprising

   (a) a carbon source selected from the group consisting of glucose, fructose, saccharose, maltose, maltodextrin, pyruvate and/or lactate, and/or
   (b) a nitrogen source selected from the group consisting of alanine, glycine, arginine and/or urea; and/or
   (c) caprylic/capric triglycerides
   for promoting the development of or stabilizing a healthy skin microbiome.

13. Non-therapeutic use of a composition comprising

   (a) a carbon source selected from the group consisting of glucose, fructose, saccharose, maltose, maltodextrin, pyruvate and/or lactate, and/or
   (b) a nitrogen source selected from the group consisting of alanine, glycine, arginine and/or urea; and/or
   (c) caprylic/capric triglycerides
   for promoting bacterial growth of the skin microbiome.

14. Non-therapeutic use of any of claims 12-13, wherein glucose is used as a carbon source in combination with alanine as a nitrogen source, and optionally caprylic/capric triglycerides.

15. Non-therapeutic use of claim 14, wherein the glucose is used in an amount of 0.5 to 3.0% (w/w) and alanine in an amount of 0.5 to 3.0% (w/w).

## Figure 1

24h growth promotion of *S.epidermidis*
n=2-4

## Figure 2

24h growth promotion of *S.epidermidis*
n=2

Figure 3 A

Figure 3 B

Figure 4

Europäisches
Patentamt

European
Patent Office

Office européen
des brevets

## EUROPEAN SEARCH REPORT

Application Number

EP 20 19 7604

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE GNPD [Online] MINTEL; 19 May 2020 (2020-05-19), anonymous: "Sym-Micro Essence", XP055776740, Database accession no. 7607177 | 1,2,4,6, 10,12,13 | INV. A61Q19/00 A61P17/10 A61P17/00 A61K8/60 A61K8/73 |
| Y | * abstract * | 1-15 | A61K8/365 A61K8/42 |
| X | US 2015/118173 A1 (FARWICK MIKE [DE] ET AL) 30 April 2015 (2015-04-30) | 1,3,4,6, 10-13 | A61K8/44 |
| Y | * claims 1,11 * * paragraphs [0003] - [0005], [0009] * * example formulation; paragraph [0069] * | 1-15 | A61K8/37 A61K8/92 A61K31/19 A61K31/17 A61K31/195 A61K31/215 |
| X | DATABASE GNPD [Online] MINTEL; 6 June 2019 (2019-06-06), anonymous: "5% Urea Nourishing Balm", XP055777715, Database accession no. 6601965 | 1,4-6, 10,12,13 | A61K31/7004 A61K31/7016 A61K31/702 |
| Y | * abstract * | 1-15 | |
| X | DATABASE GNPD [Online] MINTEL; 24 January 2018 (2018-01-24), anonymous: "Urea Plus Reparative Emollient", XP055777718, Database accession no. 5403283 | 1,5,6, 10-13 | TECHNICAL FIELDS SEARCHED (IPC) A61Q A61P A61K |
| Y | * abstract * | 1-15 | |

-/--

The present search report has been drawn up for all claims

| Place of search | Date of completion of the search | Examiner |
|---|---|---|
| Munich | 23 February 2021 | Grillenberger, Sonja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

Application Number

EP 20 19 7604

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/087640 A1 (BAUM MARC M [US]; BAUM JANELLE M [US]) 26 May 2017 (2017-05-26) | 1-3,5,8, 10-14 | |
| Y | * claims 1,3,4,5,9,10,11,13,19,20 * <br> * paragraph [0001] * <br> * paragraphs [0040] - [0043] * <br> * paragraph [0083] * <br> * examples 2-4 * | 1-15 | |
| | ----- | | |
| X | US 2019/060193 A1 (SCHMAUS GERHARD [DE] ET AL) 28 February 2019 (2019-02-28) | 1-3,6,7, 10 | |
| Y | * claims 1,2,7,9-12 * <br> * paragraph [0005] * <br> * page 16; examples A,D; table 1 * <br> * Formulation II; <br> pages 17-18; table 5 * | 1-15 | |
| | ----- | | |
| X | DATABASE GNPD [Online] <br> MINTEL; <br> 30 June 2020 (2020-06-30), <br> anonymous: "Natural Moisturizing Factors + HA", <br> XP055777721, <br> Database accession no. 7913427 | 1-6,8,10 | |
| Y | * abstract * | 1-15 | |
| | ----- | | |

**TECHNICAL FIELDS SEARCHED      (IPC)**

The present search report has been drawn up for all claims

| Place of search | Date of completion of the search | Examiner |
|---|---|---|
| Munich | 23 February 2021 | Grillenberger, Sonja |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
...........................................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 20 19 7604

This annex lists the patent family members relating to the patent documents cited in the above-mentioned European search report.
The members are as contained in the European Patent Office EDP file on
The European Patent Office is in no way liable for these particulars which are merely given for the purpose of information.

23-02-2021

| Patent document cited in search report | | Publication date | Patent family member(s) | Publication date |
|---|---|---|---|---|
| US 2015118173 | A1 | 30-04-2015 | BR 112014026906 A2 | 11-07-2017 |
| | | | CN 104284651 A | 14-01-2015 |
| | | | DE 102012206999 A1 | 31-10-2013 |
| | | | EP 2841050 A1 | 04-03-2015 |
| | | | JP 2015520735 A | 23-07-2015 |
| | | | US 2015118173 A1 | 30-04-2015 |
| | | | WO 2013160065 A1 | 31-10-2013 |
| WO 2017087640 | A1 | 26-05-2017 | US 2017143621 A1 | 25-05-2017 |
| | | | US 2019167565 A1 | 06-06-2019 |
| | | | WO 2017087640 A1 | 26-05-2017 |
| US 2019060193 | A1 | 28-02-2019 | BR 112018007384 A2 | 23-10-2018 |
| | | | CN 108472220 A | 31-08-2018 |
| | | | EP 3373895 A1 | 19-09-2018 |
| | | | JP 6784760 B2 | 11-11-2020 |
| | | | JP 2018533609 A | 15-11-2018 |
| | | | KR 20180081536 A | 16-07-2018 |
| | | | US 2019060193 A1 | 28-02-2019 |
| | | | WO 2017080625 A1 | 18-05-2017 |

EPO FORM P0459

For more details about this annex : see Official Journal of the European Patent Office, No. 12/82